# EUROPÄISCHE PATENTSCHRIFT

(11) **EP 4 447 862 B1**
(45) Veröffentlichungstag und Bekanntmachung des Hinweises auf die Patenterteilung: **09.04.2025**
(21) Anmeldenummer: 22829709.9
(22) Anmeldetag: 01.12.2022
(51) Int. Cl.: A61F 2/16

(54) **INJEKTOR MIT EINER HÜLLE MIT EINEM STOPFEN, VERPACKUNGSANORDNUNG UND VERFAHREN ZUM STERILISIEREN DES INJEKTORS**
INJECTOR HAVING A COVER WITH A STOPPER, PACKAGING ARRANGEMENT, AND METHOD FOR STERILIZING THE INJECTOR
INJECTEUR POURVU D'UN COUVERCLE DOTÉ D'UN BOUCHON, SYSTÈME D'EMBALLAGE ET PROCÉDÉ DE STÉRILISATION DE L'INJECTEUR

(30) Priorität: 17.12.2021 DE 102021133707
(43) Veröffentlichungstag der Anmeldung: 23.10.2024
(73) Patentinhaber: Carl Zeiss Meditec AG, 07745 Jena (DE)
(72) Erfinder: MOEIN, Hadi, 73447 Oberkochen (DE)
(74) Vertreter: PATERIS Patentanwälte PartmbB
(86) Internationale Anmeldenummer: PCT/EP2022/083981
(87) Internationale Veröffentlichungsnummer: WO 2023/110419

(56) Entgegenhaltungen:
- US-A- 5 616 148
- US-A1- 2007 250 068
- US-A1- 2012 071 888
- US-A1- 2020 180 800

## Beschreibung

Bei einer Kataraktbehandlung wird eine natürliche Linse eines Auges aus dem Kapselsack des Auges entfernt und durch eine künstliche Intraokularlinse ersetzt. Ein Injektor zum Einführen der Intraokularlinse in den Kapselsack kann vollständig vorgeladen sein. Dies bedeutet, dass der Injektor zusammen mit der in dem Injektor bereits angeordneten Intraokularlinse vertrieben wird. In dem Fall, dass es sich bei der Intraokularlinse um eine hydrophile Intraokularlinse handelt, ist die Intraokularlinse in dem Injektor in einer Wasser aufweisenden oder aus Wasser bestehenden Flüssigkeit zu lagern. Zum Sterilisieren des Injektors wird herkömmlich der Injektor beispielsweise in eine Verpackung eingebracht und die Verpackung gasdicht verschlossen. Die Verpackung zusammen mit dem Injektor kann anschließend in einem Autoklaven erwärmt werden. Dabei kann die Flüssigkeit an Volumen zunehmen oder zumindest teilweise verdampfen. Um zu vermeiden, dass dabei die Verpackung beschädigt wird, ist in dem Autoklaven außerhalb der Verpackung ein Druck einzustellen, der verhindert, dass die Verpackung zu weit expandiert. Jedoch kann es trotzdem vorkommen, dass Löcher und/oder Risse in der Verpackung entstehen, dass eine Delaminierung der Verpackung auftritt und/oder dass die Verpackung verknittert. In dem Fall, dass die Löcher, die Risse und/oder die Delaminierung auftritt, kann eine Kontaminierung des Injektors erfolgen. Der Injektor kann somit nicht mehr verkauft werden. Zudem haben herkömmliche Verpackungen, wie beispielsweise solche, die eine Schale und eine Aluminiumkappe oder eine Aluminiumtasche aufweisen, oftmals das Problem, dass von einem Operationspersonal eine große Kraft zum Ablösen der Aluminiumkappe oder der Aluminiumtasche aufzubringen ist.

US 2007 / 0 250 068 A1 offenbart eine Vorrichtung mit einer Verpackungsvorrichtung und einem Intraokularlinseninjektor. US 2020 / 0 180 800 A1 betrifft ein Verfahren und eine Vorrichtung zum Verpacken einer flexiblen hydrophilen Intraokularlinse.

Aufgabe der Erfindung ist es daher, einen Injektor für ein Einführen einer Intraokularlinse in den Kapselsack eines Auges, eine Verpackungsanordnung mit der Intraokularlinse und ein Verfahren zum Sterilisieren des Injektors zu schaffen, wobei der Injektor derart sterilisierbar ist, dass eine anschließende Kontamination unwahrscheinlich ist.

Der erfindungsgemäße Injektor weist ein Injektorgehäuse, das einen Gehäuseinnenraum begrenzt und eine Injektorspitze aufweist, eine Intraokularlinse, die in dem Gehäuseinnenraum angeordnet ist, einen Kolben, der längsverlagerbar entlang einer Injektorlängsachse des Injektors in dem Gehäuseinnenraum angeordnet ist und eingerichtet ist, durch ein Verlagern des Kolbens hin zu der Injektorspitze die Intraokularlinse aus dem Injektorgehäuse heraus zu schieben, und eine Hülle auf, die einen Hülleninnenraum begrenzt, in dem die Injektorspitze angeordnet ist, und eine Außenhülle aufweist, die einen ersten Zustand und einen zweiten Zustand hat, in dem die Außenhülle stärker gedehnt als in dem ersten Zustand ist, und außen an dem Injektorgehäuse anliegt sowie lediglich eine einzelne Hüllenöffnung aufweist, durch das das Injektorgehäuse sich erstreckt, wobei die Außenhülle außerhalb des Injektorgehäuses zusammen mit dem Injektorgehäuse den Hülleninnenraum abdichtet, das Injektorgehäuse ein Durchgangsloch aufweist und die Hülle sich durch das Durchgangsloch bis in den Gehäuseinnenraum erstreckt und in dem Gehäuseinnenraum einen Stopfen bildet, der innerhalb des Injektorgehäuses zusammen mit dem Injektorgehäuse den Hülleninnenraum abdichtet, wobei die Hülle eingerichtet ist, reversibel zu dehnen und somit den Hülleninnenraum zu vergrößern sowie von dem ersten Zustand in den zweiten Zustand zu gelangen, wenn der Druck innerhalb des Hülleninnenraums höher als der Druck außerhalb des Hülleninnenraums ist.

Zum Sterilisieren des Injektors kann der Injektor erwärmt werden, insbesondere in einem Autoklaven. Ein dadurch verursachter Anstieg des Drucks in dem Hülleninnenraum kann kompensiert werden, indem die Außenhülle von dem ersten Zustand in den zweiten Zustand gelangt. Dabei erfolgt auch eine Quetschung des Stopfens. Dies führt dazu, dass der Stopfen noch besser den Hülleninnenraum abdichtet. Weil die Außenhülle eingerichtet ist, reversibel zu dehnen, gelangt die Außenhülle wieder in den ersten Zustand, nachdem der Injektor wieder abgekühlt wird. Eine Beschädigung der Hülle ist dabei unwahrscheinlich. Dadurch ist auch eine Kontamination desjenigen Teils des Injektorgehäuses, das in dem Hülleninnenraum angeordnet ist, unwahrscheinlich. Anschließend kann der Injektor verschickt werden. Vor einer Durchführung einer Kataraktbehandlung ist es lediglich erforderlich, die Hülle von dem Injektorkörper abzuziehen, um den Injektor für die Kataraktbehandlung vorzubereiten. Weil die Hülle einschließlich des Stopfens eingerichtet ist, reversibel zu dehnen, kann der Stopfen dabei durch das Durchgangsloch aus dem Gehäuseinnenraum heraus gezogen werden. Die Injektorspitze kann dann in einen Kapselsack eines Auges eingesetzt werden und die Intraokularlinse kann durch ein Verlagern des Kolbens zu der Injektorspitze hin aus dem Injektorgehäuse heraus und somit in den Kapselsack hinein geschoben werden. Die Hülle kann beispielsweise einen Elastizitätsmodul in einem Bereich von 0,1 MPa bis 5 MPa haben.

Es ist bevorzugt, dass in dem Gehäuseinnenraum in Richtung der Injektorlängsachse zwischen der Injektorspitze und dem Stopfen eine Flüssigkeit, die insbesondere Wasser aufweist, angeordnet ist. Bei der Flüssigkeit kann es sich beispielsweise um Wasser, insbesondere demineralisiertes Wasser, eine physiologische Kochsalzlösung und/oder ein Viskoelastikum (OVD, englisch: ophthalmic viscoelasic device) handeln. Weil das Volumen der Flüssigkeit bei einem Erwärmen verdampfen kann, ist eine besonders große Volumenzunahme des Hülleninnenraums zu erwarten, so dass es besonders vorteilhaft ist, die Hülle zum Kompensieren der Volumenzunahme vorzusehen.

Zudem ist es vorteilhaft nicht erforderlich, vor der Kataraktbehandlung die Intraokularlinse mit einer Flüssigkeit zu benetzen.

Die Intraokularlinse ist bevorzugt eine hydrophile Intraokularlinse. Die hydrophile Intraokularlinse eignet sich besonders dazu, in einem Injektor gelagert werden, weil bei einer eventuell während einer Lagerung auftretenden Quetschung der Intraokularlinse diese aufgrund ihrer guten elastischen Eigenschaften nach der Lagerung wieder ihre ursprüngliche Form annimmt.

Die Hülle weist bevorzugt Silikon auf oder besteht bevorzugt aus dem Silikon. Silikon hat eine gute thermische Beständigkeit, so dass keine Beschädigung der Hülle auftritt, wenn der Injektor erwärmt wird. Zudem hat Silikon gute elastische Eigenschaften, so dass auch keine Beschädigung der Hülle auftritt, wenn die Außenhülle von dem ersten Zustand in den zweiten Zustand und zurück in den ersten Zustand gelangt.

Das Injektorgehäuse weist bevorzugt eine außenliegende Gehäusedichtfläche auf, die sich vollumfänglich um das Injektorgehäuse erstreckt und beabstandet von der Injektorspitze angeordnet ist, und die Außenhülle weist bevorzugt eine Hüllendichtfläche auf, die die Gehäusedichtfläche vollumfänglich kontaktiert, wodurch der Hülleninnenraum außerhalb des Injektorgehäuses abgedichtet ist. Dabei ist besonders bevorzugt, dass die Hülle in Richtung der Injektorlängsachse ein erstes Hüllenlängsende, das in Richtung der Injektorlängsachse in einem Bereich des Injektorgehäuses angeordnet ist, und ein zweites Hüllenlängsende aufweist, das in Richtung der Injektorlängsachse in einem Bereich außerhalb des Injektorgehäuses angeordnet ist, wobei die Hüllendichtfläche in einem Bereich angeordnet ist, der sich in dem ersten Zustand in Richtung der Injektorlängsachse ausgehend von dem ersten Hüllenlängsende bis zu maximal 20 %, insbesondere bis zu maximal 10 %, eines Abstands von dem ersten Hüllenlängsende zu dem zweiten Hüllenlängsende erstreckt. Dadurch kann der Hülleninnenraum besonders groß ausgeführt werden.

Es ist bevorzugt, dass die Außenhülle mittels einer in einem Bereich der Hüllendichtfläche angeordneten Presspassung an dem Injektorgehäuse, mittels mindestens einer Schraube und/oder mittels eines in einem Bereich der Hüllendichtfläche angeordneten Befestigungsrings, der außerhalb der Außenhülle angeordnet ist und die Außenhülle an dem Injektorgehäuse festklemmt, befestigt ist.

Der Stopfen hat bevorzugt einen ersten Querschnitt und einen zweiten Querschnitt, deren Normalen parallel zu der Injektorlängsachse angeordnet sind, wobei der erste Querschnitt näher als der zweite Querschnitt an der Injektorspitze angeordnet ist und kleiner als der zweite Querschnitt ist. Dies ist besonders relevant, wenn der Stopfen in einem sich in Richtung zu der Injektorspitze hin verjüngenden Bereich des Gehäuseinnenraums angeordnet ist, weil dadurch eine Anpassung der Form des Stopfens an die Form des Gehäuseinnenraums erfolgen kann, wodurch eine besonders gute Abdichtung des Hülleninnenraums resultiert. Dabei ist besonders bevorzugt, dass der Stopfen die Form eines Kegelstumpfes hat.

Es ist bevorzugt, dass der Stopfen ein erstes Stopfenlängsende, das der Intraokularlinse abgewandt angeordnet ist, und ein zweites Stopfenlängsende aufweist, das der Intraokularlinse zugewandt angeordnet ist, und die Hülle ein Verbindungsstück aufweist, das sich durch das Durchgangsloch erstreckt und in Richtung der Injektorlängsachse beabstandet von dem zweiten Stopfenlängsende angeordnet ist. Der Stopfen ist von dem Verbindungsstück festgehalten. Wenn der Druck innerhalb des Hülleninnenraums höher als der Druck außerhalb des Hülleninnenraums wird, wird dadurch der Stopfen insbesondere in einem Bereich gequetscht, der sich von dem zweiten Stopfenlängsende bis zu dem Verbindungsstück erstreckt.

Das Injektorgehäuse weist bevorzugt einen Injektorkörper und eine Kartusche auf, die lösbar mit dem Injektorkörper gekoppelt ist und in der die Intraokularlinse angeordnet ist. Dabei ist besonders bevorzugt, dass die Gehäusedichtfläche von dem Injektorkörper gebildet ist.

Die erfindungsgemäße Verpackungsanordnung weist eine Verpackung und den Injektor auf, der in der Verpackung angeordnet ist.

Das erfindungsgemäße Verfahren zum Sterilisieren des Injektors weist den Schritt auf: - Erwärmen des Injektors. Die Temperatur des Injektors kann dabei beispielsweise bis zu 132 °C betragen und insbesondere für eine Zeit von maximal 30 Minuten gehalten werden. Die Temperatur kann mindestens 40 °C, insbesondere mindestens 60 °C betragen. Der Druck in einem außerhalb an die Hülle angrenzenden Bereich kann beispielsweise höher als Atmosphärendruck sein. Die Atmosphäre in dem außerhalb an die Hülle angrenzenden Bereich kann beispielsweise sauerstofffrei sein.

Der Injektor wird bevorzugt in einer Verpackung angeordnet und zusammen mit der Verpackung erwärmt. Dadurch wird vorteilhaft erreicht, dass auch die Verpackung sterilisiert wird. Die Verpackung kann beispielsweise eingerichtet sein, eine Übertragung von Wärme und Feuchtigkeit von außerhalb der Verpackung nach innerhalb der Verpackung zu erlauben. Eine solche Verpackung ist beispielsweise unter dem Namen Medipeel kommerziell erhältlich.

Im Folgenden wird anhand der beigefügten schematischen Zeichnungen die Erfindung näher erläutert. Es zeigen
Figur 1 einen erfindungsgemäßen Injektor mit einer Außenhülle in einem ersten Zustand und
Figur 2 den Injektor aus Figur 1 mit der Außenhülle in einem zweiten Zustand.

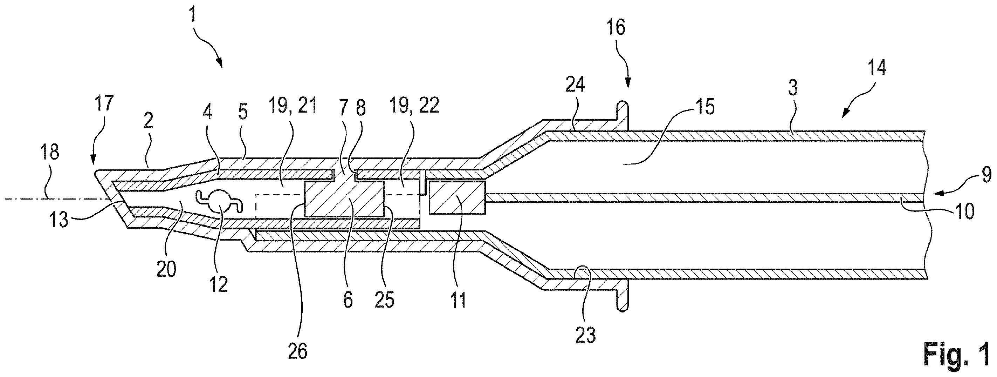

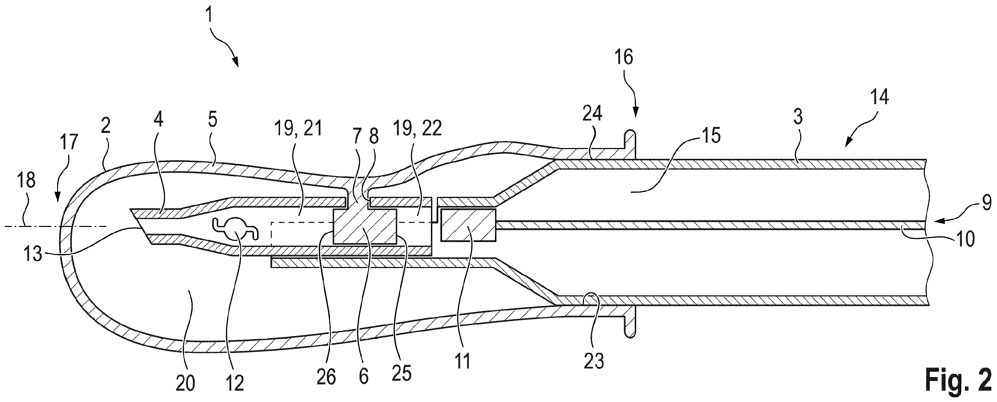

Wie es aus Figuren 1 und 2 ersichtlich ist, weist ein Injektor 1 ein Injektorgehäuse 14, eine Intraokularlinse 12, einen Kolben 9 und eine Hülle 2 auf. Das Injektorgehäuse 14 begrenzt einen Gehäuseinnenraum 19 und weist eine Injektorspitze 13 auf. Die Intraokularlinse 12 ist in dem Gehäuseinnenraum 19 angeordnet. Der Kolben 9 ist längsverlagerbar entlang einer Injektorlängsachse 18 des Injektors 1 in dem Gehäuseinnenraum 19 angeordnet und eingerichtet, durch ein Verlagern des Kolbens 9 hin zu der Injektorspitze 13 die Intraokularlinse 12 aus dem Injektorgehäuse 14 heraus zu schieben. Die Hülle 2 begrenzt einen Hülleninnenraum 20, wobei die Injektorspitze 13 in dem Hülleninnenraum 20 angeordnet ist. Zudem weist die Hülle 2 eine Außenhülle 5 auf, die einen ersten Zustand und einen zweiten Zustand hat, in dem die Außenhülle 5 stärker als in dem ersten Zustand gedehnt ist, und außen an dem Injektorgehäuse 14 anliegt sowie lediglich eine einzelne Hüllenöffnung 15 aufweist, durch das das Injektorgehäuse 14 sich erstreckt. Die Außenhülle 5 dichtet außerhalb des Injektorgehäuses 14 zusammen mit dem Injektorgehäuse 14 den Hülleninnenraum 20 ab. Das Injektorgehäuse 14 weist ein Durchgangsloch 8 auf und die Hülle 2 erstreckt sich durch das Durchgangsloch 8 bis in den Gehäuseinnenraum 19 und bildet in dem Gehäuseinnenraum 19 einen Stopfen 6. Der Stopfen 6 dichtet innerhalb des Injektorgehäuses 14 zusammen mit dem Injektorgehäuse 14 den Hülleninnenraum 20 ab. Die Hülle 2 ist eingerichtet, reversibel zu dehnen und somit den Hülleninnenraum 20 zu vergrößern sowie von dem ersten Zustand in den zweiten Zustand zu gelangen, wenn der Druck innerhalb des Hülleninnenraums 20 höher als der Druck außerhalb des Hülleninnenraums 20 ist.

Die Hülle 2 kann auch ein Verbindungsstück 7 aufweisen, das sich durch das Durchgangsloch 8 erstreckt und fest mit der Außenhülle 5 und dem Stopfen 6 verbunden ist. Der Stopfen 6 kann eingerichtet sein, den Gehäuseinnenraum 19 in einen ersten Teilinnenraum 21, der ein Teil des Hülleninnenraums 20 ist, und einen zweiten Teilinnenraum 22 zu teilen, die in Richtung der Injektorlängsachse 18 nebeneinander angeordnet sind. Dadurch, dass der Stopfen 6 innerhalb des Gehäuseinnenraums 19 den Hülleninnraum 20 abdichtet, kann somit kein Fluid von dem ersten Teilinnenraum 21 in den zweiten Teilinnenraum 22 und umgekehrt gelangen.

Die Hülle 2 kann beispielsweise eingerichtet sein, derart reversibel zu dehnen, dass das Volumen des Hülleninnenraums 20 in dem zweiten Zustand mindestens 10 %, insbesondere mindestens 15 %, größer als in dem ersten Zustand ist. Die Hülle 2 kann beispielsweisen einen Elastizitätsmodul in einem Bereich von 0,1 MPa bis 5 MPa haben. Dies gilt für die Außenhülle 5 und insbesondere auch für den Stopfen 6 und/oder das Verbindungsstück 7. Die Außenhülle 5, der Stopfen 6 und das Verbindungsstück 7 können einstückig und aus dem gleichen Werkstoff ausgebildet sein.

Figur 1 zeigt den Injektor 1 in dem ersten Zustand der Außenhülle 5 und Figur 2 zeigt den Injektor 1 in dem zweiten Zustand der Außenhülle 5. Durch ein Erwärmen des Injektors 1 kann sich ein in dem Injektorgehäuse 14 zwischen der Injektorspitze 13 und dem Stopfen 6 angeordnetes Fluid ausdehnen und durch die Injektorspitze 13 nach außerhalb des Injektorgehäuses 14 gelangen, wodurch die Außenhülle 5 gedehnt wird. Dadurch kann es vorkommen, dass es Bereiche gibt, in denen in dem ersten Zustand die Außenhülle 5 an dem Injektorgehäuse 14 anliegt und in dem zweiten Zustand von dem Injektorgehäuse 14 beabstandet angeordnet ist, vergleiche Figuren 1 und 2. Es ist auch denkbar, dass das Fluid via einen in dem Durchgangsloch 8 angeordneten Spalt zwischen dem Injektorgehäuse 14 und dem Verbindungsstück 7 nach außerhalb des Injektorgehäuses 14 gelangt. Um den Injektor 1 zu erwärmen, kann der Injektor 1 beispielsweise in einen Autoklaven eingebracht werden. Insbesondere kann dabei der Injektor 1 in einer Verpackung angeordnet sein. Die Temperatur des Injektors 1 kann dabei beispielsweise von 40 °C oder von 60 °C bis 132 °C betragen und insbesondere für eine Zeit von maximal 30 Minuten gehalten werden. Der Druck in einem außerhalb an die Hülle angrenzenden Bereich kann beispielsweise höher als der Atmosphärendruck sein. Die Atmosphäre in dem außerhalb an die Hülle angrenzenden Bereich kann beispielsweise sauerstofffrei sein.

In dem Gehäuseinnenraum 19 kann in Richtung der Injektorlängsachse 18 zwischen der Injektorspitze 13 und dem Stopfen 6 eine Flüssigkeit, die insbesondere Wasser aufweist, angeordnet sein. Bei der Flüssigkeit kann es sich beispielsweise um Wasser, insbesondere demineralisiertes Wasser, eine physiologische Kochsalzlösung und/oder ein Viskoelastikum (OVD, englisch: ophthalmic viscoelasic device) handeln. Die Intraokularlinse 12 kann eine hydrophile Intraokularlinse 12 sein. Die Intraokularlinse 12 kann in einem gefalteten Zustand oder in einem ungefalteten Zustand in dem Injektorgehäuse 14 angeordnet sein.

Figuren 1 und 2 zeigen, dass das Injektorgehäuse 14 eine außenliegende Gehäusedichtfläche 24 aufweisen kann, die sich vollumfänglich um das Injektorgehäuse 14 erstreckt und beabstandet von der Injektorspitze 13 angeordnet ist, und die Außenhülle 5 kann eine Hüllendichtfläche 23 aufweisen, die die Gehäusedichtfläche 24 vollumfänglich kontaktiert, wodurch der Hülleninnenraum 20 außerhalb des Injektorgehäuses 14 abgedichtet ist. Der Hüllenraum 20 ist dabei sowohl in dem ersten Zustand als auch in dem zweiten Zustand außerhalb des Injektorgehäuses 14 durch die Hüllendichtfläche 23 und die Gehäusedichtfläche 24 abgedichtet. Die Hülle 2 kann in Richtung der Injektorlängsachse 18 ein erstes Hüllenlängsende 16, das in Richtung der Injektorlängsachse 18 in einem Bereich des Injektorgehäuses 14 angeordnet ist und an dem die Hüllenöffnung 15 angeordnet sein kann, und ein zweites Hüllenlängsende 17 aufweisen, das in Richtung der Injektorlängsachse 18 in einem Bereich außerhalb des Injektorgehäuses 14 angeordnet ist, wobei die Hüllendichtfläche 23 in einem Bereich angeordnet sein kann, der sich in dem ersten Zustand in Richtung der Injektorlängsachse 18 ausgehend von dem ersten Hüllenlängsende 16 bis zu maximal 20 % oder 10 % eines Abstands von dem ersten Hüllenlängsende 16 zu dem zweiten Hüllenlängsende 17 erstreckt. Die Außenhülle 5 kann mittels einer in einem Bereich der Hüllendichtfläche 23 angeordneten Presspassung an dem Injektorgehäuse 14, mittels mindestens einer Schraube und/oder mittels eines in einem Bereich der Hüllendichtfläche 23 angeordneten Befestigungsrings, der außerhalb der Außenhülle 5 angeordnet ist und die Außenhülle 5 an dem Injektorgehäuse 14 festklemmt, befestigt sein. Die Presspassung hat zur Folge, dass die Außenhülle 5 in dem Bereich der Hüllendichtfläche 23 sowohl in dem zweiten Zustand als auch in dem ersten Zustand unter einer Zugspannung steht. Es ist denkbar, dass in dem ersten Zustand die Außenhülle 5 ungedehnt ist. In dem Fall, dass die Presspassung vorgesehen ist, bedeutet dies, dass in dem ersten Zustand die Außenhülle 5 lediglich in dem Bereich der Hüllendichtfläche 23 aufgrund der Presspassung gedehnt ist. Alternativ dazu, dass in dem ersten Zustand die Außenhülle 5 ungedehnt ist, kann in dem ersten Zustand die Außenhülle 5 auch gedehnt sein, jedoch weniger stark als in dem zweiten Zustand.

Es ist denkbar, dass gemäß einer nicht in Figuren 1 und 2 dargestellten Ausführungsform der Stopfen 6 einen ersten Querschnitt und einen zweiten Querschnitt hat, deren Normalen parallel zu der Injektorlängsachse 18 angeordnet sind, wobei der erste Querschnitt näher als der zweite Querschnitt an der Injektorspitze 13 angeordnet ist und kleiner als der zweite Querschnitt ist. Der Stopfen 6 kann beispielsweise die Form eines Kegelstumpfes haben.

Wie es aus Figuren 1 und 2 ersichtlich ist, kann der Stopfen 6 ein erstes Stopfenlängsende 25, das der Intraokularlinse 12 abgewandt angeordnet ist, und ein zweites Stopfenlängsende 26 aufweisen, das der Intraokularlinse 12 zugewandt angeordnet ist, und die Hülle 2 kann ein Verbindungsstück 7 aufweisen, das sich durch das Durchgangsloch 8 erstreckt und in Richtung der Injektorlängsachse 18 beabstandet von dem zweiten Stopfenlängsende 26 angeordnet ist. Zudem kann das Verbindungsstück 7 in Richtung der Injektorlängsachse 18 beabstandet von dem ersten Stopfenlängsende 25 angeordnet sein. Die Figuren zeigen, dass in dem zweiten Zustand der Stopfen in einem Bereich zwischen dem zweiten Stopfenlängsende 26 und dem Verbindungsstück 7 gequetscht ist.

Figuren 1 und 2 zeigen, dass das Injektorgehäuse 14 einen Injektorkörper 3 und eine Kartusche 4 aufweisen kann, die lösbar mit dem Injektorkörper 3 gekoppelt ist und in der die Intraokularlinse 12 angeordnet ist. Das Durchgangsloch 8 kann in der Kartusche 4 angeordnet sein. Die Gehäusedichtfläche 24 kann von dem Injektorkörper 3 gebildet sein. Der Kolben 9 kann innerhalb des Injektorkörpers 3 angeordnet sein.

Der Kolben 9 kann eine Kolbenstange 10 und einen Kissen 11 aufweisen, das an dem der Injektorspitze 13 zugewandten Längsende der Kolbenstange 10 angeordnet ist. Das Kissen 11 kann eingerichtet sein, gequetscht zu werden, wenn es in den verjüngenden Bereich des Gehäuseinnenraums 19 gelangt.

### Bezugszeichenliste

1 Injektor
2 Hülle
3 Injektorkörper
4 Kartusche
5 Außenhülle
6 Stopfen
7 Verbindungsstück
8 Durchgangsloch
9 Kolben
10 Kolbenstange
11 Kissen
12 Intraokularlinse
13 Injektorspitze
14 Injektorgehäuse
15 Hüllenöffnung
16 erstes Hüllenlängsende
17 zweites Hüllenlängsende
18 Injektorlängsachse
19 Gehäuseinnenraum
20 Hülleninnenraum
21 erster Teilinnenraum
22 zweiter Teilinnenraum
23 Hüllendichtfläche
24 Gehäusedichtfläche
25 erstes Stopfenlängsende
26 zweites Stopfenlängsende

## Patentansprüche

1. Injektor mit einem Injektorgehäuse (14), das einen Gehäuseinnenraum (19) begrenzt und eine Injektorspitze (13) aufweist, einer Intraokularlinse (12), die in dem Gehäuseinnenraum (19) angeordnet ist, einem Kolben (9), der längsverlagerbar entlang einer Injektorlängsachse (18) des Injektors (1) in dem Gehäuseinnenraum (19) angeordnet ist und eingerichtet ist, durch ein Verlagern des Kolbens (9) hin zu der Injektorspitze (13) die Intraokularlinse (12) aus dem Injektorgehäuse (14) heraus zu schieben, und einer Hülle (2), die einen Hülleninnenraum (20) begrenzt, in dem die Injektorspitze (13) angeordnet ist, und eine Außenhülle (5) aufweist, die außen an dem Injektorgehäuse (14) anliegt sowie lediglich eine einzelne Hüllenöffnung (15) aufweist, durch das das Injektorgehäuse (14) sich erstreckt, wobei die Außenhülle (5) außerhalb des Injektorgehäuses (14) zusammen mit dem Injektorgehäuse (14) den Hülleninnenraum (20) abdichtet, **dadurch gekennzeichnet, dass** die Außenhülle (5) einen ersten Zustand und einen zweiten Zustand hat, in dem die Außenhülle (5) stärker als in dem ersten Zustand gedehnt ist, das Injektorgehäuse (14) ein Durchgangsloch (8) aufweist und die Hülle (2) sich durch das Durchgangsloch (8) bis in den Gehäuseinnenraum (19) erstreckt und in dem Gehäuseinnenraum (19) einen Stopfen (6) bildet, der innerhalb des Injektorgehäuses (14) zusammen mit dem Injektorgehäuse (14) den Hülleninnenraum (20) abdichtet, wobei die Hülle (2) eingerichtet ist, reversibel zu dehnen und somit den Hülleninnenraum (20) zu vergrößern sowie von dem ersten Zustand in den zweiten Zustand zu gelangen, wenn der Druck innerhalb des Hülleninnenraums (20) höher als der Druck außerhalb des Hülleninnenraums (20) ist.

2. Injektor gemäß Anspruch 1, wobei in dem Gehäuseinnenraum (19) in Richtung der Injektorlängsachse (18) zwischen der Injektorspitze (13) und dem Stopfen (6) eine Flüssigkeit, die insbesondere Wasser aufweist, angeordnet ist, insbesondere wobei die Intraokularlinse (12) eine hydrophile Intraokularlinse (12) ist.

3. Injektor gemäß Anspruch 1 oder 2, wobei das Injektorgehäuse (14) eine außenliegende Gehäusedichtfläche (24) aufweist, die sich vollumfänglich um das Injektorgehäuse (14) erstreckt und beabstandet von der Injektorspitze (13) angeordnet ist, und die Außenhülle (5) eine Hüllendichtfläche (23) aufweist, die die Gehäusedichtfläche (24) vollumfänglich kontaktiert, wodurch der Hülleninnenraum (20) außerhalb des Injektorgehäuses (14) abgedichtet ist.

4. Injektor gemäß Anspruch 3, wobei die Hülle (2) in Richtung der Injektorlängsachse (18) ein erstes Hüllenlängsende (16), das in Richtung der Injektorlängsachse (18) in einem Bereich des Injektorgehäuses (14) angeordnet ist, und ein zweites Hüllenlängsende (17) aufweist, das in Richtung der Injektorlängsachse (18) in einem Bereich außerhalb des Injektorgehäuses (14) angeordnet ist, wobei die Hüllendichtfläche (23) in einem Bereich angeordnet ist, der sich in dem ersten Zustand in Richtung der Injektorlängsachse (18) ausgehend von dem ersten Hüllenlängsende (16) bis zu maximal 20 % eines Abstands von dem ersten Hüllenlängsende (16) zu dem zweiten Hüllenlängsende (17) erstreckt.

5. Injektor gemäß Anspruch 3 oder 4, wobei die Außenhülle (5) mittels einer in einem Bereich der Hüllendichtfläche (23) angeordneten Presspassung an dem Injektorgehäuse (14), mittels mindestens einer Schraube und/oder mittels eines in einem Bereich der Hüllendichtfläche (23) angeordneten Befestigungsrings, der außerhalb der Außenhülle (5) angeordnet ist und die Außenhülle (5) an dem Injektorgehäuse (14) festklemmt, befestigt ist.

6. Injektor gemäß einem der Ansprüche 1 bis 5, wobei der Stopfen (6) einen ersten Querschnitt und einen zweiten Querschnitt hat, deren Normalen parallel zu der Injektorlängsachse (18) angeordnet sind, wobei der erste Querschnitt näher als der zweite Querschnitt an der Injektorspitze (13) angeordnet ist und kleiner als der zweite Querschnitt ist, wobei der Stopfen (6) insbesondere die Form eines Kegelstumpfes hat.

7. Injektor gemäß einem der Ansprüche 1 bis 6, wobei der Stopfen (6) ein erstes Stopfenlängsende (25), das der Intraokularlinse (12) abgewandt angeordnet ist, und ein zweites Stopfenlängsende (26) aufweist, das der Intraokularlinse (12) zugewandt angeordnet ist, und die Hülle (2) ein Verbindungsstück (7) aufweist, das sich durch das Durchgangsloch (8) erstreckt und in Richtung der Injektorlängsachse (18) beabstandet von dem zweiten Stopfenlängsende (26) angeordnet ist.

8. Injektor gemäß einem der Ansprüche 1 bis 7, wobei das Injektorgehäuse (14) einen Injektorkörper (3) und eine Kartusche (4) aufweist, die lösbar mit dem Injektorkörper (3) gekoppelt ist und in der die Intraokularlinse (12) angeordnet ist.

9. Verpackungsanordnung mit einer Verpackung und einem Injektor (1) gemäß einem der Ansprüche 1 bis 8, der in der Verpackung angeordnet ist.

10. Verfahren zum Sterilisieren eines Injektors (1) gemäß einem der Ansprüche 1 bis 8, mit dem Schritt: Erwärmen des Injektors (1) gemäss einem der Ansprüche 1 bis 8.

11. Verfahren gemäss Anspruch 10, wobei der Injektor (1) in einer Verpackung angeordnet wird und zusammen mit der Verpackung erwärmt wird.

## Claims

1. Injector having an injector housing (14) which delimits a housing interior (19) and has an injector tip (13), an intraocular lens (12) which is arranged in the housing interior (19), a piston (9) which is arranged longitudinally displaceably along an injector longitudinal axis (18) of the injector (1) in the housing interior (19) and which, by a displacement of the piston (9) towards the injector tip (13), is designed to push the intraocular lens (12) out of the injector housing (14), and a sleeve (2) which delimits a sleeve interior (20), in which the injector tip (13) is arranged, and has an outer sleeve (5) which bears on the outside of the injector housing (14) and has only a single sleeve opening (15) through which the injector housing (14) extends, wherein the outer sleeve (5) outside the injector housing (14), together with the injector housing (14), seals off the sleeve interior (20), **characterized in that** the outer sleeve (5) has a first state and a second state in which the outer sleeve (5) is stretched further than in the first state, the injector housing (14) has a through-hole (8), and the sleeve (2) extends through the through-hole (8) into the housing interior (19) and forms a stopper (6) in the housing interior (19), which stopper, inside the injector housing (14), and together with the injector housing (14), seals off the sleeve interior (20), wherein the sleeve (2) is designed to stretch reversibly and thus enlarge the sleeve interior (20) and pass from the first state to the second state when the pressure inside the sleeve interior (20) is higher than the pressure outside the sleeve interior (20).

2. Injector according to Claim 1, wherein a liquid, which in particular contains water, is arranged in the housing interior (19), between the injector tip (13) and the stopper (6) in the direction of the injector longitudinal axis (18), in particular wherein the intraocular lens (12) is a hydrophilic intraocular lens (12).

3. Injector according to Claim 1 or 2, wherein the injector housing (14) has an external housing sealing surface (24) which extends around the entire circumference of the injector housing (14) and is arranged spaced apart from the injector tip (13), and the outer sleeve (5) has a sleeve sealing surface (23) which contacts the housing sealing surface (24) over the entire circumference, as a result of which the sleeve interior (20) is sealed off outside the injector housing (14).

4. Injector according to Claim 3, wherein the sleeve (2) has, in the direction of the injector longitudinal axis (18), a first sleeve longitudinal end (16) which, in the direction of the injector longitudinal axis (18), is arranged in a region of the injector housing (14), and a second sleeve longitudinal end (17) which, in the direction of the injector longitudinal axis (18), is arranged in a region outside of the injector housing (14), wherein the sleeve sealing surface (23) is arranged in a region which, in the first state, and in the direction of the injector longitudinal axis (18), extends from the first sleeve longitudinal end (16) up to a maximum of 20% of a distance from the first sleeve longitudinal end (16) to the second sleeve longitudinal end (17).

5. Injector according to Claim 3 or 4, wherein the outer sleeve (5) is fastened, by means of an interference fit arranged in a region of the sleeve sealing surface (23), on the injector housing (14), by means of at least one screw and/or by means of a fastening ring arranged in a region of the sleeve sealing surface (23), which fastening ring is arranged outside the outer sleeve (5) and clamps the outer sleeve (5) to the injector housing (14).

6. Injector according to any one of Claims 1 to 5, wherein the stopper (6) has a first cross section and a second cross section, of which the normals are arranged parallel to the injector longitudinal axis (18), wherein the first cross section is closer than the second cross section to the injector tip (13) and is smaller than the second cross section, wherein the stopper (6) has in particular the shape of a truncated cone.

7. Injector according to any one of Claims 1 to 6, wherein the stopper (6) has a first stopper longitudinal end (25), which is arranged facing away from the intraocular lens (12), and a second stopper longitudinal end (26), which is arranged facing the intraocular lens (12), and the sleeve (2) has a connecting piece (7) which extends through the through-hole (8) and, in the direction of the injector longitudinal axis (18), is arranged spaced apart from the second stopper longitudinal end (26).

8. Injector according to any one of Claims 1 to 7, wherein the injector housing (14) has an injector body (3) and a cartridge (4) which is releasably coupled to the injector body (3) and in which the intraocular lens (12) is arranged.

9. Packaging arrangement comprising a package and an injector (1) according to any one of Claims 1 to 8, which injector is arranged in the package.

10. Method for sterilizing an injector (1) according to any of Claims 1 to 8, comprising the step of: heating the injector (1) according to any one of Claims 1 to 8.

11. Method according to Claim 10, wherein the injector (1) is arranged in a package and is heated together with the package.

## Revendications

1. Injecteur comportant un corps intégral (14) d'injecteur, qui limite un espace intérieur (19) de corps intégral et comporte une pointe (13) d'injecteur, une lentille intraoculaire (12) qui est disposée dans l'espace intérieur (19) de corps intégral, un piston (9) qui est disposé dans l'espace intérieur (19) de corps intégral, apte à se déplacer longitudinalement le long d'un axe longitudinal (18) d'injecteur de l'injecteur (1) et est conçu pour pousser la lentille intraoculaire (12) hors du corps intégral (14) d'injecteur vers la pointe (13) d'injecteur par un déplacement du piston (9), et comporte une enveloppe (2) qui limite un espace interne (20) d'enveloppe, dans lequel est disposée la pointe (13) d'injecteur, et une enveloppe externe (5) qui est appliquée extérieurement sur le corps intégral (14) d'injecteur et présente uniquement une seule ouverture (15) d'enveloppe, à travers laquelle s'étend le corps intégral (14) d'injecteur, l'enveloppe externe (5) étanchéifiant à l'extérieur du corps intégral (14) d'injecteur, conjointement avec le corps intégral (14) d'injecteur, l'espace intérieur (20) de l'enveloppe, **caractérisé en ce que** l'enveloppe externe (5) présente un premier état et un second état dans lequel l'enveloppe externe (5) est plus fortement étirée que dans le premier état, le corps intégral (14) d'injecteur comporte un trou de passage (8) et l'enveloppe (2) s'étend à travers le trou de passage (8) jusque dans l'espace interne (19) de corps intégral et forme dans l'espace interne (19) de corps intégral un bouchon (6) qui, conjointement avec le corps intégral (14) d'injecteur, étanchéifie l'espace interne (20) de corps intégral à l'intérieur du corps intégral (14) d'enveloppe, l'enveloppe (2) étant conçue pour s'expanser réversiblement et agrandir ainsi l'espace interne (20) d'enveloppe ainsi que pour passer du premier état au second état, lorsque la pression à l'intérieur de l'espace interne (20) d'enveloppe est plus élevée que la pression à l'extérieur de l'espace interne (20) d'enveloppe.

2. Injecteur selon la revendication 1, dans lequel un liquide, qui comporte en particulier de l'eau, est disposé dans l'espace interne (19) de corps intégral entre, en direction de l'axe longitudinal (18) d'injecteur, la pointe (13) d'injecteur et le bouchon (6), la lentille intraoculaire (12) étant en particulier une lentille intraoculaire hydrophile (12).

3. Injecteur selon la revendication 1 ou 2, dans lequel le corps intégral (14) d'injecteur présente une surface d'étanchéité (24) externe de corps intégral, qui s'étend sur toute la circonférence autour du corps intégral (14) d'injecteur et est disposée à distance de la pointe (13) d'injecteur, et l'enveloppe externe (5) présente une surface d'étanchéité (23) d'enveloppe, qui est en contact sur toute la circonférence avec la surface d'étanchéité (24) de corps intégral, ce par quoi l'espace interne (20) d'enveloppe est étanchéifié à l'extérieur du corps intégral (14) d'injecteur.

4. Injecteur selon la revendication 3, dans lequel l'enveloppe (2) présente en direction de l'axe longitudinal (18) d'injecteur une première extrémité longitudinale (16) d'enveloppe, qui en direction de l'axe longitudinal (18) d'injecteur est disposée dans une zone du corps intégral (14) d'injecteur, et une seconde extrémité longitudinale (17) d'enveloppe, qui est disposée en direction de l'axe longitudinal (18) d'injecteur dans une zone à l'extérieur du corps intégral (14) d'injecteur, la surface d'étanchéité (23) d'enveloppe s'étendant dans le premier état en direction de l'axe longitudinal (18) d'injecteur à partir de la première extrémité longitudinale (16) d'enveloppe jusqu'à 20 % au maximum d'une distance allant de la première extrémité longitudinale (16) d'enveloppe à la seconde extrémité longitudinale (17) d'enveloppe.

5. Injecteur selon la revendication 3 ou 4, dans lequel l'enveloppe externe (5) est fixée au corps intégral (14) d'injecteur au moyen d'un ajustement serré disposé dans une zone de la surface d'étanchéité (23) d'enveloppe, au moyen d'au moins une vis et/ou au moyen d'une bague de fixation disposée dans une zone de la surface d'étanchéité (23) d'enveloppe, qui est disposée à l'extérieur de l'enveloppe externe (5) et serre étroitement l'enveloppe externe (5) sur le corps intégral (14) d'injecteur.

6. Injecteur selon l'une quelconque des revendications 1 à 5, dans lequel le bouchon (6) a une première section transversale et une seconde section transversale, dont les normales sont disposées parallèlement à l'axe longitudinal (18) d'injecteur, la première section transversale étant disposée plus près de la pointe (13) d'injecteur que la seconde section transversale et étant plus petite que la seconde section transversale, le bouchon (6) ayant en particulier une forme d'un tronc de cône.

7. Injecteur selon l'une quelconque des revendications 1 à 6, dans lequel le bouchon (6) présente une première extrémité longitudinale (25) de bouchon qui est disposée à l'opposé de la lentille intraoculaire (12), et une seconde extrémité longitudinale (26) de bouchon qui est disposée en face de la lentille intraoculaire (12), et l'enveloppe (2) comporte une pièce de liaison (7) qui s'étend à travers le trou de passage (8) et est disposée en étant espacée, en direction de l'axe longitudinal (18) d'injecteur, de la seconde extrémité longitudinale (26) de bouchon.

8. Injecteur selon l'une quelconque des revendications 1 à 7, dans lequel le corps intégral (14) d'injecteur comporte un corps (3) d'injecteur et une cartouche (4) qui est accouplée de façon amovible au corps (3) d'injecteur et dans laquelle est disposée la lentille intraoculaire (12).

9. Ensemble d'emballage comportant un emballage et un injecteur (1) selon l'une quelconque des revendications 1 à 8, qui est disposé dans l'emballage.

10. Procédé pour la stérilisation d'un injecteur (1) selon l'une quelconque des revendications 1 à 8, comportant l'étape : chauffage de l'injecteur (1) selon l'une quelconque des revendications 1 à 8.

11. Procédé selon la revendication 10, dans lequel l'injecteur (1) est disposé dans un emballage et chauffé conjointement avec l'emballage.
